# EUROPÄISCHE PATENTSCHRIFT

(11) **EP 2 475 419 B1**
(45) Veröffentlichungstag und Bekanntmachung des Hinweises auf die Patenterteilung: **12.04.2017**
(21) Anmeldenummer: 10779204.6
(22) Anmeldetag: 30.08.2010
(51) Int. Cl.: A61N 1/05, A61N 1/36, A61N 1/39, A61B 17/34

(54) **SAKRALER NEUROMODULATOR**
SACRAL NEUROMODULATOR
DISPOSITIF DE NEUROMODULATION SACRÉE

(30) Priorität: 11.09.2009 DE 102009040963
(43) Veröffentlichungstag der Anmeldung: 18.07.2012
(73) Patentinhaber: Sievert, Karl-Dietrich, 5020 Salzburg (AT)
(72) Erfinder: Sievert, Karl-Dietrich, 5020 Salzburg (AT)
(86) Internationale Anmeldenummer: PCT/DE2010/001000
(87) Internationale Veröffentlichungsnummer: WO 2011/029419

(56) Entgegenhaltungen:
- GB-A- 2 048 682
- US-A- 6 165 180
- US-A1- 2003 233 126
- US-A1- 2004 210 245
- US-A1- 2006 004 421
- US-A1- 2007 100 411
- US-A1- 2007 173 900
- US-A1- 2008 132 970
- US-A1- 2009 012 592
- US-B2- 6 999 819

## Beschreibung

Gegenstand der Erfindung ist eine Stimulationsvorrichtung mit mindestens einer Stimulationselektrode (unilateral) bzw. Stimulationselektroden (bilateral) nach dem Oberbegriff des Anspruchs 1 sowie ein Introducer-Kit.

Miktions-, Defäkations und Sexualfunktionsstörungen sind Folgen einer Schädigung der zentralen autonomen Zentren der spinalen sympathischen und parasympathischen Kerngebiete oder der peripheren autonomen Ganglien und Nerven.
Im Bereich der Miktionsstörungen, also der Störungen des Harnabgabe-, Blasensystems sind elektrische Stimulationsvorrichtungen wie beispielsweise in der US 2006/0190047 A1 beschrieben, bekannt. Derartige Stimulationsvorrichtungen werden bisher bereits bei Miktionsstörungen anderer Genere eingesetzt. Für Patienten mit Querschnittsläsion und neurogener Blasenentleerungsstörungen werden dagegen lediglich medikamentöse Therapien oder weit invasivere Maßnahmen angeboten. Vorteilhaft ist jedoch ihre minimalinvasive Implantation. Elektroden vergleichbarer Gattung sind beispielsweise auch aus der US 2004 021 0245 A1 bekannt.
Unter einem spinalen Querschnittsyndrom, ein Synonym für Querschnittlähmung, Querschnittläsion, Transversalsyndrom, wird ein Komplex von Symptomen verstanden, die bei Unterbrechung der Nervenleitung im Rückenmark auftreten. Die Ursache können Verletzungen des Rückenmarks, z. B. bei Wirbelbrüchen, aber auch Tumore und andere spezielle Erkrankungen, z. B. Multiple Sklerose sein. Hierbei liegt eine neuronale Dysfunktion ab dem Bereich der Schädigung vor, welche beispielsweise im Bereich des Urogenitaltraktes zur Inkontinenz/Problemen bei der Harnentleerung, einer Detrusor-Sphinker-Dyssynergie (DSD) sowie zu einem Verlust von Sexualfunktionen sowie der Stuhlentleerung (Fehlfunktion des kleinen Beckens) führen kann.

Das schwierigste Ereignis, die DSD führt zu einer gestörten Koordination der für eine normale Harnblasenfunktion benötigten Muskulatur mit Restharnbildung und einem gesteigerten intravesikalen Druck bei der Blasenentleerung. Dies kann zu Detrusorschädigung, vesikoureteralem Reflux und damit letztlich zu einer Nierenschädigung führen.

Zusätzlich kann bei einem spinalen Querschnittsyndrom auch die sexuelle Funktionalität, insbesondere bei männlichen Patienten aufgrund der fehlenden Erektionsfähigkeit stark beeinträchtigt sein.

Eine bekannte implantierbare Elektrode zur Stimulation ist aus der DE 698 25 588 T2 bekannt.

Bisherige minimalinvasive Therapien nutzen Stimulationselektroden, welche an geeigneten Stellen implantiert werden, und die erforderliche Stimulation der neuronalen Strukturen unterstützen/übernehmen. Die Implantation wird hierbei individuell auf das erfolgreichste Stimulationsergebnis abgestimmt, welches wiederum erheblich von der Position der Elektrode bezüglich der zu stimulierenden Muskulatur abhängt.

Problematisch ist hierbei, dass sich die Elektroden zu einem späteren Zeitpunkt in Ihrer Position verschieben können, da eine Bewegung des Körpers und des Gewebes, welches die Elektrode hält, erfolgt. Die präzise und abgestimmte Positionierung geht hierbei verloren.

Aus dem Stand der Technik sind Stimulationselektroden sowie Kanülen zur gezielten Führung eines Objekts im Körper, beispielsweise aus US 6999819B2, US2009012592 A1, US 2007173900 A1, US 2006004421 A1, US 2004210245 A1, US 2003233126 A1, GB 2048682 A, US 2008&132970 A1 und US 6165180 A bekannt.

Aus der US 2007100411 A1 ist eine Elektrode bekannt, welche Vorschub und Rückzug verhindernde Haltemittel aufweist. Nachteilig an dieser Ausführung ist, dass die Position nicht sofort nach dem Positionieren in beide Richtungen längs der Elektrode fixiert ist, sondern dass zuerst der Abbau eines Bandes erfolgen muss, welches die Vorschub verhindernden Mittel blockiert.

### Aufgabe der Erfindung:

Aufgabe der Erfindung ist es, eine Vorrichtung zur Positionierung und zum Positionserhalt von Stimulationselektroden und ein Verfahren zur einheitlichen Programmierung und Steuerung der Stimulationselektroden und zum Erhalt der Miktionsfähigkeit bei Patienten mit spinalem Querschnittsyndrom, insbesondere mit einer Detrusor-Sphinker-Dyssynergie, und somit zur Erzeugung einer kompletten Kontinenz bei normaler urodynamischer Kapazität zur Verfügung zu stellen.

Dies bezieht sich auch auf stuhlregulierende Effekte, d.h. Beeinflussung der Dickdarmfunktion.

Diese Aufgabe wird durch eine Positionierungs- und Positionserhaltungsvorrichtung für Stimulationselektroden nach Anspruch 1 gelöst. In den Unteransprüchen sind vorteilhafte Weiterbildungen sowie zweckmäßige Ausgestaltungen angegeben.

Zur genaueren Betrachtung der vorliegenden Erfindung ist in der Erfindungsbeschreibung ein Verweis auf die jeweiligen Bezugszeichen des in den Figuren dargestellten Ausführungsbeispiels gegeben.

Hierbei zeigen:

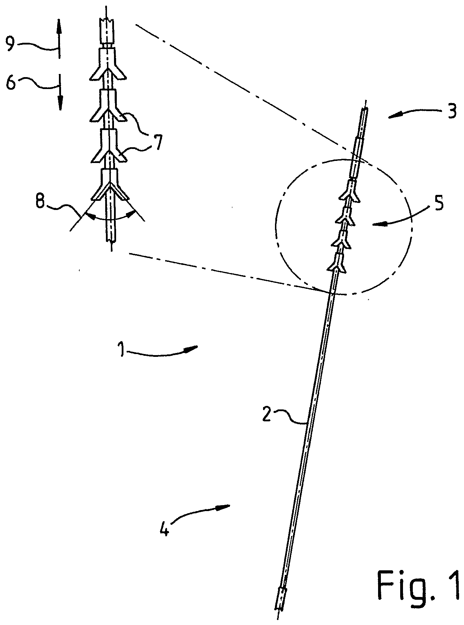

- Fig.1: Eine Stimulationselektrode (1) nach dem Stand der Technik

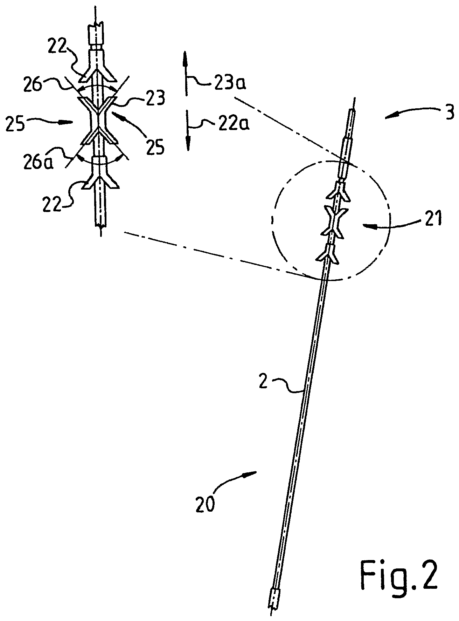

- Fig.2: Eine erfindungsgemäße Stimulationselektrode (20)

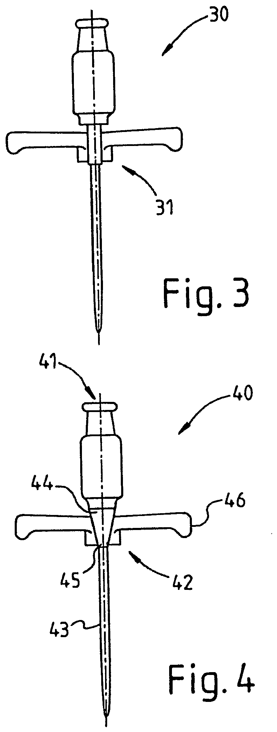

- Fig.3: Ein Introducer-Kit (30) nach dem Stand der Technik
- Fig.4: Ein erfindungsgemäßes Introducer Kit (40)
- Fig.5: a) Eine Elektrode nach dem Stand der Technik
b) Eine erfindungsgemäße Elektrode

Erfindungsgemäß ist die Stimulationselektrode (20) für eine Stimulationsvorrichtung zur Stimulation von Organen bei spinalem Querschnittsyndrom, insbesondere bei Detrusor-Sphinker-Dyssynergie und Fehlfunktion von Organen im kleinen Becken, umfassend einen Elektrodenschaft (2), an dessen einen Ende eine Elektrodenspitze (3) zur Abgabe eines Stimulationssignals angeordnet ist, und an dessen anderem Ende ein Anschlussbereich (4) für eine Signalleitung vorgesehen ist ausgebildet. Die Fixierung sollte hierbei an der Elektrode nahe an den Elektrodenpunkten angeordnet sein, damit - auch für Patienten mit Querschnitt, die in dieser Lokalisation Muskel- bzw. Unterhautfettgewebe abbauen - eine bessere Fixierung und Orientierung zur zu stimulierenden Nervenwurzel möglich ist (ca. 1/3 geringer als die herkömmliche Distanz zwischen proximaler Elektrode und distalem Haken). Sie umfasst weiterhin mindestens ein Fixierungsmittel (5,21) zur Fixierung der Position des Elektrodenschafts (2) im umgebenden Gewebe entlang seiner Längsachse wobei dieses dadurch gekennzeichnet ist, dass das Fixierungsmittel (21) je mindestens ein Rückzug in Rückzugsrichtung(22a) und mindstens ein Vorschub in Vorschubrichtung (23a) verhinderndes Haltemittel (22,23) aufweisend ausgebildet ist.

Hierdurch wird der Erhalt der exakten Position gewährleistet.

Im Stand der Technik ist nur die Rückzugsrichtung (6) durch Haltemittel (7) mit einem Hakenwinkel (8) gesichert. Die Vorschubrichtung (9) erfährt keine Widerhaken.

Eine Weiterbildung ist eine Stimulationselektrode (20),dadurch gekennzeichnet, dass das Rückzug verhindernde Haltemittel (22) und das Vorschub verhindernde Haltemittel (23) durch einen Klemmbereich (24) beabstandet und mit diesem verbunden sind, wobei der Klemmbereich (24) eine kraftschlüssige Verbindung zwischen Elektrodenschaft (2) und den Haltemitteln (22,23) herstellt.

In einer weiteren Ausführung der Stimulationselektrode ist an der Verbindung zwischen Klemmbereich (24) und Haltemittel (22,23) ein Knickbereich (25), vorzugsweise eine Einschnürung zur Verschwenkung des Haltemittels beim Überschreiten einer Schwellenkraft vorgesehen.

Auf diese Weise kann die Elektrode trotz der vorhandenen Haltemittel herausgezogen werden, wenn eine ausreichende Zugkraft angewandt wird. Die Haltemittel klappen dabei um und stellen nur noch einen stark verminderten Widerstand gegen eine Bewegung dar.

In einer weiteren Form der Stimulationselektrode ist diese dadurch gekennzeichnet, dass die Haltemittel (22,23) durch vom Elektrodenschaft (2) unter einem Hakenwinkel (26,26a) weggerichtete Widerhaken gebildet werden.

Eine Ausführung der Stimulationselektrode sieht vor, dass das Rückzug verhindernden Haltemittel (22) symmetrisch zu einer den Elektrodenschaft (2) senkrecht schneidenden Spiegelebene gegenüber dem Vorschub verhindernden Haltemittel (23) ausgebildet ist.
Auf diese Weise wird in beide Vorschubrichtungen (22a,23a) eine vergleichbare Haltewirkung erzielt.

Eine weitere Ausführungsform der Stimulationselektrode beinhaltet, dass die Haltemittel (22,23) durch in einem Öffnungswinkel (26,26a) auf den Elektrodenschaft zulaufende Spreizelemente gebildet werden, wobei die Winkelspitze entgegen der jeweiligen Bewegungsrichtung (22a, 23a) gerichtet ist, in welche das Haltemittel (22,23) den Vorschub verhindert.

Weiterhin umfasst die Erfindung eine Einführungshilfe, genannt "Introducer-Kit" (40) für eine erfindungsgemäße Stimulationselektrode (20), welches einen Elektrodeneingang (41) zur Einführung des Elektrodenschafts (2) in das Introducer-Kit (40), eine Führung (42) zur Inaktivierung der Haltemittel (22,23) während des Positioniervorgangs der Elektrode (20) und eine Kanüle (43) zur Durchdringung von Gewebe umfasst und dadurch gekennzeichnet ist, dass die Führung (42) konisch ausgebildet ist.

Durch die konische Führung werden die Haltemittel an den Elektrodenschaft angelegt und in die Kanüle eingeführt. Erst nach dem Verlassen der Kanüle können die Haltemittel wieder beispielsweise ihren Hakenwinkel (26,26a) einnehmen, und somit den gewünschten Halt im Gewebe bereitstellen.

In einer Weiterbildung ist das erfindungsgemäße Introducer-Kit dadurch gekennzeichnet, dass die konische Führung mit einem erweiterten Öffnungsbereich (44) an der Seite des Elektrodeneingangs (41), und mit einem demgegenüber verkleinerten Öffnungsbereich (45) an der Seite der Kanüle (43) angeordnet ist.

Vorteilhafter Weise ist am Introducer-Kit eine Handhabungshilfe (46) vorgesehen.

Ein Introducer-Kit (30) nach dem Stand der Technik weist nur einen zylindrisch geradlinigen Führungsbereich (31) auf, welcher insbesondere keine Einwirkung auf Vorschub hindernde Haltemittel ausüben kann. Diese wären mit dem Introducer-Kit (30) nach dem Stand der Technik nicht anwendbar.

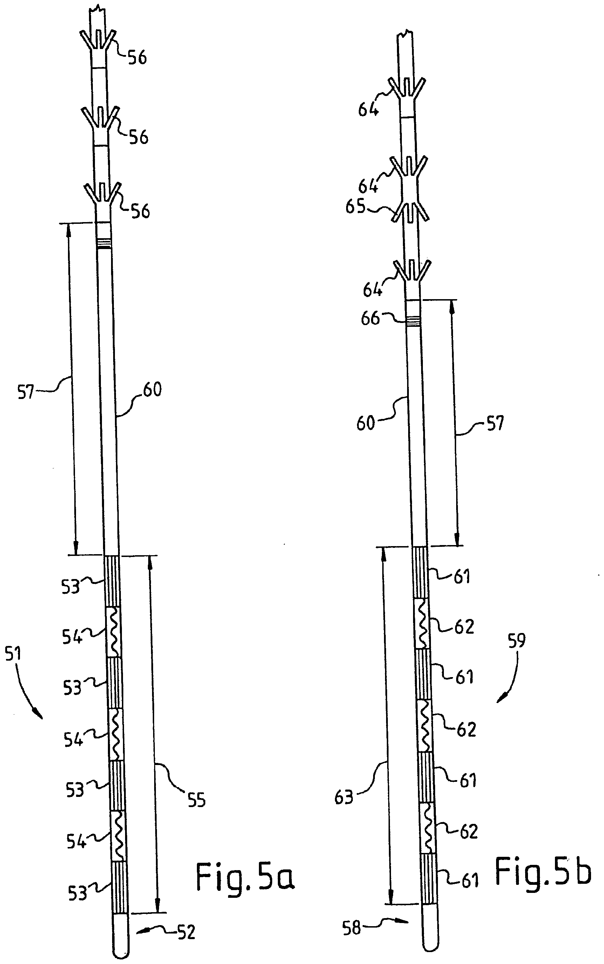

Eine weitere zweckmäßige Ausgestaltung der erfindungsgemäßen Stimulationselektrode ist in Fig. 5 a) und b) dargestellt. Figur 5 a) zeigt eine Elektrode nach dem Stand der Technik. Die Elektrodenspitze 51 umfasst ein vorderes Spitzenende 52, welche das Eindringen in das Gewebe erleichtert. Auf das Spitzenende folgend befinden sich am Elektrodenschaft angeordnete Kontaktbereiche 53 (Elektrodenringe), welche durch Isolationsbereiche 54 voneinander beabstandet sind. Auf diese Weise wird an der Elektrodenspitze 51 ein Stimulationsbereich 55 mit mehreren Elektrodenringen 53 ausgebildet, welcher in direkten Kontakt mit dem/den Nerv/en treten kann.

Nachfolgend auf den Stimulationsbereich 55 folgt in ca. 8mm Abstand 57 eine Anordnung von Widerhaken 56, welche als Rückzugverhinderndes Haltemittel eingesetzt werden.

Figur 5 b) zeigt eine erfindungsgemäße Stimulationselektrode mit einem vorderen Spitzenende 58 an der Elektrodenspitze 59, auf welche nachfolgend am Elektrodenschaft 60 im Abstand von ca. 3mm Elektrodenringe 61 angeordnet sind. Die Elektrodenringe 61 selbst sind durch Zwischenbereiche 62 voneinander beabstandet, wobei der Abstand jeweils ebenfalls ca. 3mm beträgt. In diesem Stimulationsbereich 63 der Anordnung der Elektrodenringe 61 findet eine Stimulation der/des Nerven/s statt.

Der Stimulationsbereich 63 ist vom Bereich der Fixierungsmittel mit Rückzug verhindernden Haltemitteln (Widerhaken in Zugrichtung) 64 und Vorschub verhindernden Haltemitteln (Widerhaken in Schubrichtung) 65 um einen Abstand von ca. 4-6 mm, vorzugsweise 5 mm entlang des Elektrodenschafts 60 versetzt. Die dichtere Positionierung der Fixierungsmittel erreicht eine verbesserte Positionsstabilität der implantierten Stimulationselektrode und damit ein verbessertes Wirkergebnis. Zusätzlich sind am Elektrodenschaft noch einer oder mehrere Markierungen 66 vorgesehen, welche die Eindringtiefe insbesondere beim Applizierungsvorgang der Elektrode kennzeichnen.

Exemplarisch ist das Verfahren zur Stimulation von Organen bei spinalem Querschnittsyndrom, insbesondere bei Detrusor-Sphinker Dyssynergie, mittels vorzugsweise 2 oder 4 erfindungsgemäßen Stimulationselektroden auszuführen. Dabei wird in einem ersten Verfahrensschritt ein Softstart der Stimulation über einen ersten kurzen Zeitraum, der "Start-Zeit" ausgeführt. In einem zweiten Verfahrensschritt erfolgt ein abwechselndes Einschalten für eine "AnZeit" und anschließendes Abschalten für eine "Aus-Zeit" der Stimulation, wobei die Intensität des Stimulationssignals im zweiten Verfahrensschritt sowie das Verhältnis von "An-Zeit" zur "Aus-Zeit" als wählbare Programmparameter zur Verfügung stehen. Beispielhaft hat sich die "Start-Zeit" im Bereich von 1s - 30s, vorzugsweise bei 8s als vorteilhaft herausgestellt. Weiterhin liegt die vorteilhafte "An-Zeit" in einem ersten Programm im Bereich von 10 min - 60 min, vorzugsweise bei 29 min.
Darüber hinaus ist es vorteilhaft, dass die "Aus-Zeit" im Bereich von 5 min - 40 min liegt, vorzugsweise 15 min beträgt.

### Bezugszeichenliste

- 1: Elektrode (Stand der Technik)
- 2: Elektrodenschaft
- 3: Elektrodenspitze
- 4: Anschlußbereich
- 5: Fixierungsmittel
- 6: Rückzugsrichtung
- 7: Rückzug verhinderndes Haltemittel
- 8: Hakenwinkel
- 9: Vorschubrichtung
- 20: Elektrode
- 21: Fixierungsmittel
- 22: Rückzug verhinderndes Haltemittel
- 22a: Rückzugsrichtung
- 23: Vorschub verhinderndes Haltemittel
- 23a: Vorschubrichtung
- 24: Klemmbereich
- 25: Knickbereich
- 26: Hakenwinkel (Vorschub)
- 26a: Hakenwinkel (Rückzug)
- 30: Introducer-Kit (Stand der Technik)
- 31: Führung
- 40: Introducer-Kit
- 41: Elektrodeneingang
- 42: Führung
- 43: Kanüle
- 44: erweiterter Öffnungsbereich
- 45: verkleinerter Öffnungsbereich
- 46: Handhabungshilfe
- 51: Elektrodenspitze
- 52: vorderes Spitzenende
- 53: Kontaktbereich
- 54: Isolationsbereich
- 55: Stimulationsbereich
- 56: Widerhaken
- 57: Abstand
- 58: Spitzenende
- 59: Elektrodenspitze
- 60: Elektrodenschaft
- 61: Elektrodenringe
- 62: Zwischenbereich
- 63: Stimulationsbereich
- 64: Widerhaken in Zugrichtung
- 65: Widerhaken in Schubrichtung
- 66: Markierung

## Patentansprüche

1. Positionierungs- und Positionserhaltungsvorrichtung für Stimulationselektroden wobei die Vorrichtung eine Stimulationselektrode für eine Stimulationsvorrichtung zur Stimulation von Organen bei spinalem Querschnittsyndrom, insbesondere bei Detrusor-Sphinker-Dyssynergie umfasst und
- die Stimulationselektrode einen Elektrodenschaft, an dessen einem Ende eine Elektrodenspitze zur Abgabe eines Stimulationssignals angeordnet ist, und an dessen anderem Ende ein Anschlussbereich für eine Signalleitung vorgesehen ist aufweist, und
- die Stimulationselektrode mindestens ein Fixierungsmittel zur Fixierung der Position des Elektrodenschafts im umgebenden Gewebe entlang seiner Längsachse aufweist,
- wobei das Fixierungsmittel je mindestens ein Rückzug verhinderndes Haltemittel und mindestens ein Vorschub verhinderndes Haltemittel aufweisend ausgebildet ist
- wobei dass das Rückzug und das Vorschub verhindernde Haltemittel durch einen Klemmbereich beabstandet und mit diesem verbunden sind, wobei der Klemmbereich eine kraftschlüssige Verbindung zwischen Elektrodenschaft und den Haltemitteln herstellt
- und an der Verbindung zwischen Klemmbereich und Haltemittel ein Knickbereich, vorzugsweise eine Einschnürung zur Verschwenkung des Haltemittels beim überschreiten einer Schwellenkraft vorgesehen ist
und
b) eine Einführungshilfe für die Stimulationselektrode umfasst, wobei die Einführhilfe
- einen Elektrodeneingang zur Einführung des Elektrodenschafts in die Einführhilfe und
- eine konisch ausgebildete Führung zur Inaktivierung der Haltemittel während des Positioniervorgangs der Elektrode aufweist und
- eine Kanüle zur Durchdringung von Gewebe umfasst.

2. Positionierungs- und Positionserhaltungsvorrichtung nach Anspruch 1, **dadurch gekennzeichnet, dass** die Haltemittel durch vom Elektrodenschaft unter einem Hakenwinkel weggerichtete Widerhaken gebildet werden.

3. Positionierungs- und Positionserhaltungsvorrichtung nach einem der vorhergegangenen Ansprüche, **dadurch gekennzeichnet, dass** das Rückzug verhindernden Haltemittel symmetrisch zu einer den Elektrodenschaft senkrecht schneidenden Spiegelebene gegenüber dem Vorschub verhindernden Haltemittel ausgebildet ist.

4. Positionierungs- und Positionserhaltungsvorrichtung nach einem der vorhergegangenen Ansprüche, **dadurch gekennzeichnet, dass** die Haltemittel durch in einem Öffnungswinkel auf den Elektrodenschaft zulaufende Spreizelemente gebildet werden, wobei die Winkelspitze entgegen der jeweiligen Bewegungsrichtung gerichtet ist, in welche das Haltemittel den Vorschub verhindert.

5. Positionierungs- und Positionserhaltungsvorrichtung nach einem der vorangegangenen Ansprüche, **dadurch gekennzeichnet, dass** an der Elektrodenspitze zwischen einem vorderen Spitzenende und einem Bereich mit Fixierungsmitteln Elektrodenringe für einen Kontakt zu Nerven vorgesehen sind, wobei die Elektrodenringe vorzugsweise eine Länge und/oder einen Abstand von ca. 2,5mm bis 3,5mm, insbesondere 3mm zueinander aufweisen und wobei zwischen Fixierungsmittel und Elektrodenringen ein Abstand von 4 mm bis 6 mm vorgesehen ist.

6. Positionierungs- und Positionserhaltungsvorrichtung einem der vorangegangenen Ansprüche, **dadurch gekennzeichnet, dass** die konische Führung mit einem erweiterten Öffnungsbereich an der Seite des Elektrodeneingangs, und mit einem demgegenüber verkleinerten Öffnungsbereich an der Seite der Kanüle angeordnet ist.

7. Positionierungs- und Positionserhaltungsvorrichtung nach einem der vorangegangenen Ansprüche, **dadurch gekennzeichnet**, eine Handhabungshilfe an der Einführungshilfe vorgesehen ist.

## Claims

1. Positioning and position-maintaining apparatus for stimulation electrodes, wherein the apparatus comprises a stimulation electrode for a stimulation apparatus for stimulating organs in the case of paraplegia, in particular detrusor sphincter dyssynergia, and
- the stimulation electrode has an electrode shaft, at one end of which an electrode tip is arranged for emitting a stimulation signal and at the other end of which a connection region is provided for a signal line, and
- the stimulation electrode has at least one fixing means for fixing the position of the electrode shaft in the surrounding tissue along the longitudinal axis of said electrode shaft,
- wherein the fixing means in each case is embodied having at least one holding means preventing a withdrawal and at least one holding means preventing an advance,
- wherein the holding means preventing the withdrawal and the holding means preventing the advance are spaced apart by a clamping region and connected thereto, the clamping region establishing a force-fit connection between electrode shaft and the holding means, and
- a kink region is provided on the connection between clamping region and holding means, preferably a constriction for pivoting the holding means if a threshold force is exceeded,
and
b) comprises an insertion aid for the stimulation electrode, wherein the insertion aid has
- an electrode entrance for introducing the electrode shaft into the insertion aid and
- a guide with a conical design for inactivating the holding means during the positioning procedure of the electrode, and
- comprises a cannula for penetrating tissue.

2. Positioning and position-maintaining apparatus according to Claim 1, **characterized in that** the holding means are formed by barbed hooks directed away from the electrode shaft at a hook angle.

3. Positioning and position-maintaining apparatus according to one of the preceding claims, **characterized in that** the holding means preventing a withdrawal is designed symmetrically to a mirror plane intersecting the electrode shaft perpandicularly, with respect to the holding means preventing an advance.

4. Positioning and position-maintaining apparatus according to one of the preceding claims, **characterized in that** the holding means are formed by expanding elements that taper toward the electrode shaft at an opening angle, the tip of the angle being directed counter to the respective movement direction in which the holding means prevents the advance.

5. Positioning and position-maintaining apparatus according to one of the preceding claims, **characterized in that**, on the electrode tip between a front tip end and a region with fixing means, provision is made for electrode rings for contact with nerves, the electrode rings preferably having a length and/or a distance from one another of approximately 2.5 mm to 3.5 mm, more particularly 3 mm, and a distance of 4 mm to 6 mm being provided between fixing means and electrode rings.

6. Positioning and position-maintaining apparatus according to one of the preceding claims, **characterized in that** the conical guide is arranged such that a widened opening region is on the side of the electrode entrance and a reduced opening region compared thereto is on the side of the cannula.

7. Positioning and position-maintaining apparatus according to one of the preceding claims, **characterized in that** provision is made for a handling aid on the insertion aid.

## Revendications

1. Dispositif de positionnement et de maintien de position destiné à des électrodes de stimulation, dans lequel le dispositif comprend une électrode de stimulation destinée à un dispositif de stimulation pour stimuler des organes dans le cas d'un syndrome quadriplégique spinal, notamment dans le cas d'une dyssynergie entre muscle expulseur et sphincter et
- l'électrode de stimulation comporte un arbre d'électrode sur une extrémité duquel est disposé une pointe d'électrode destinée à délivrer un signal de stimulation, et sur l'autre extrémité duquel est prévue une zone de raccordement destinée à une ligne de signal, et
- l'électrode de stimulation comporte au moins un moyen de fixation destiné à fixer la position de l'arbre d'électrode dans le tissu environnant le long de son axe longitudinal,
- dans lequel le moyen de fixation est respectivement réalisé à partir d'au moins un moyen de maintien empêchant le déplacement vers l'arrière et d'au moins un moyen de maintien empêchant le déplacement vers l'avant,
- dans lequel les moyens de maintien empêchant le déplacement vers l'arrière et vers l'avant sont espacés par une région de serrage et sont reliés à celle-ci, dans lequel la région de serrage établit une liaison par complémentarité de force entre l'arbre d'électrode et les moyens de maintien,
- et il est prévu au niveau de la liaison entre la région de serrage et les moyens de maintien une partie coudée, de préférence un rétrécissement, destinée à faire pivoter le moyen de maintien lorsqu'un seuil de force est dépassé, et
b) comprend un accessoire d'insertion destiné à l'électrode de stimulation, dans lequel l'accessoire d'insertion comporte
- une entrée d'électrode destinée à l'insertion de l'arbre d'électrode dans l'accessoire d'insertion, et
- un guide de forme conique destiné à désactiver le moyen de maintien pendant le processus de positionnement de l'électrode et
- une canule destinée à traverser des tissus.

2. Dispositif de positionnement et de maintien de position selon la revendication 1, **caractérisé en ce que** les moyens de maintien sont formés par des picots partant de l'arbre d'électrode en formant un angle de picot.

3. Dispositif de positionnement et de maintien de position selon l'une quelconque des revendications précédentes, **caractérisé en ce que** le moyen de maintien empêchant le déplacement vers l'arrière est réalisé de manière symétrique du moyen de maintien empêchant le déplacement vers l'avant par rapport à un plan miroir coupant perpendiculairement l'arbre d'électrode.

4. Dispositif de positionnement et de maintien de position selon l'une quelconque des revendications précédentes, **caractérisé en ce que** les moyens de maintien sont formés par des éléments d'écartement se terminant par un angle d'ouverture sur l'arbre d'électrode, dans lequel le sommet est orienté en sens opposé à la direction de déplacement respective dans laquelle le moyen de maintien empêche le déplacement vers l'avant.

5. Dispositif de positionnement et de maintien de position selon l'une quelconque des revendications précédentes, **caractérisé en ce qu'**il est prévu des électrodes annulaires permettant un contact avec des nerfs sur les pointes d'électrodes entre l'extrémité de pointe avant et une région comportant des moyens de fixation, dans lequel les électrodes annulaires présentent de préférence une longueur et/ou un espacement d'environ 2,5 mm à 3,5 mm, en particulier, de 3 mm l'une par rapport à l'autre, et dans lequel il est prévu entre les moyens de fixation et les électrodes annulaires un espacement de 4 mm à 6 mm.

6. Dispositif de positionnement et de maintien de position selon l'une quelconque des revendications précédentes, **caractérisé en ce que** le guide conique est disposé de manière à comporter une région d'ouverture élargie du côté de l'entrée d'électrode, et de manière à comporter une région d'ouverture réduite par rapport à celle-ci du côté de la canule.

7. Dispositif de positionnement et de maintien de position selon l'une quelconque des revendications précédentes, **caractérisé en ce qu'**il est prévu un accessoire de manipulation sur l'accessoire d'introduction.
